# EUROPEAN PATENT APPLICATION

(11) **EP 3 327 921 A1**
(43) Date of publication of application: **30.05.2018**
(21) Application number: 17202618.9
(22) Date of filing: 20.11.2017
(51) Int. Cl.: H02M 7/797, H03K 17/687, H03K 17/567, H01L 25/16, H05G 1/32, H02M 3/335

(54) **HYBRID SWITCH FOR INVERTER OF COMPUTED TOMOGRAPHY SYSTEM**

(30) Priority: 23.11.2016 US 201615359850
(71) Applicant: General Electric Company, Schenectady, NY 12345 (US)
(72) Inventor: LOUVRIER, Yannick, 78530 Buc (FR); ROBERT, Christophe, 78530 Buc (FR); ERNEST, Philippe, 78530 Buc (FR); LEVILLY, Nicolas, 78530 Buc (FR)
(74) Representative: Bedford, Grant Richard

(57) **Abstract**

An inverter 54 for a computed tomography (CT) system 10 is provided. The inverter 54 includes a hybrid switch 64. The hybrid switch 64 includes a silicon carbide metal-oxide-semiconductor field-effect transistor (SiC MOSFET) portion 66, an insulated-gate bipolar transistor (IGBT) portion 68, a first gate 70 associated within the SiC MOSFET portion 66, and a second gate 72 associated with the IGBT portion 68. The SiC MOSFET portion 66 and the IGBT portion 68 of the hybrid switch 64 are configured to be independently controlled via the first gate 70 and the second gate 72.

## Description

The subject matter disclosed herein relates generally to a medical imaging system and, in particular, to a hybrid switch for an inverter of a computed tomography system.

Typically, in computed tomography (CT) imaging systems, an X-ray source emits a fan or cone-shaped beam toward a subject or object, such as a patient or a piece of luggage. Hereinafter, the terms "subject" and "object" shall include anything capable of being imaged. The beam, after being attenuated by the subject, impinges upon an array of radiation detectors. The intensity of the attenuated beam radiation received at the detector array is typically dependent upon the attenuation of the x-ray beam by the subject. Each detector element of the detector array produces a separate electrical signal indicative of the attenuated beam received by each detector element. The electrical signals are transmitted to a data processing system for analysis which ultimately produces an image.

Generally, the X-ray source and the detector array are rotated about the gantry within an imaging plane and around the subject. X-ray sources typically include X-ray tubes, which emit the X-ray beam at a focal point. X-ray detectors typically include a collimator for collimating X-ray beams received at the detector, a scintillator for converting X-rays to light energy adjacent the collimator, and photodiodes for receiving the light energy from the adjacent scintillator and producing electrical signals therefrom. Typically, each scintillator of a scintillator array converts X-rays to light energy. Each scintillator discharges light energy to a photodiode adjacent thereto. Each photodiode detects the light energy and generates a corresponding electrical signal. The outputs of the photodiodes are transmitted to the data processing system for image reconstruction. Imaging data may be obtained using X-rays that are generated at a single polychromatic energy. However, some systems may obtain multi-energy images that provide additional information for generating images, and therefore include fast switching of X-ray tube kV between two discrete levels of, for instance, 80 and 140 kV. And, it is generally desired to have a crisp transition between the high and low frequencies and if the switching is too slow, blurring can occur in reconstructed images.

The X-ray generator of a CT system is typically located within the gantry and, as such, rotates about an imaging bore during data acquisition on a rotatable side of the gantry. The X-ray generator includes the X-ray source, a high voltage (HV) tank, and an inverter. On the stationary side of the gantry is a power distribution unit (PDU). The inverter is typically fed with a DC voltage and generates an AC waveform. The AC voltage is fed to the HV tank, which has a transformer and rectifiers that develop a DC HV potential. The HV potential is applied to the X-ray source.

According to one known configuration, the inverter is positioned on the rotating base and therefore rotates with data acquisition components. The inverter includes switches that switch in a pattern to control the inverter current, and thus output power of the converter. The switching between on and off is controlled in such a fashion that a highfrequency inverter current is formed, which is in turn fed to the HV tank, as stated. However as detector scintillator technology and other system operating parameters have increased, so too has the need to operate at a higher frequency. Current switching technology has limited power converter performance.

Certain embodiments commensurate in scope with the originally claimed subject matter are summarized below. These embodiments are not intended to limit the scope of the claimed subject matter, but rather these embodiments are intended only to provide a brief summary of possible forms of the subject matter. Indeed, the subject matter may encompass a variety of forms that may be similar to or different from the embodiments set forth below.

In one embodiment, an X-ray generation system is provided. The X-ray generation system includes an X-ray source, a high voltage tank coupled to the X-ray source, and an inverter coupled to the high voltage tank. The inverter includes a hybrid switch that includes a silicon carbide metal-oxide-semiconductor field-effect transistor (SiC MOSFET) portion and an insulated-gate bipolar transistor (IGBT) portion. The X-ray generation system also include a controller programed to control commutation of the hybrid switch based on a frequency and a power utilized by the X-ray generation system.

In an additional embodiment, a method for utilizing an inverter of a computed tomography (CT) system is provided. The method includes determining, via a controller, a power and a frequency for operation of the inverter, wherein the inverter includes a hybrid switch that includes a silicon carbide metal-oxide-semiconductor field-effect transistor (SiC MOSFET) portion and an insulated-gate bipolar transistor (IGBT) portion. The SiC MOSFET portion has a lower current rating than the IGBT portion. The method also include independently controlling, via the controller, which portion of the hybrid switch to utilize based on the power and the frequency.

In a further embodiment, an inverter for a computed tomography (CT) system is provided. The inverter includes a hybrid switch. The hybrid switch includes a silicon carbide metal-oxide-semiconductor field-effect transistor (SiC MOSFET) portion, an insulated-gate bipolar transistor (IGBT) portion, a first gate associated within the SiC MOSFET portion, and a second gate associated with the IGBT portion. The SiC MOSFET portion and the IGBT portion of the hybrid switch are configured to be independently controlled via the first gate and the second gate.

Various features, aspects, and advantages of the present subject matter will become better understood when the following detailed description is read with reference to the accompanying drawings in which like characters represent like parts throughout the drawings, wherein:
FIG. 1 is a combined pictorial view and block diagram of a CT imaging system illustrating an embodiment of the present disclosure;
FIG. 2 is a schematic diagram of a portion of the CT imaging system of FIG. 1;
FIG. 3 is a circuit diagram of an embodiment of a hybrid switch for an inverter of the CT imaging system of FIGS. 1 and 2;
FIG. 4 is a flow chart of a method for utilizing a hybrid switch of an inverter for a CT imaging system; and
FIG. 5 is a graph of timing for commutation of a hybrid switch in accordance with an embodiment of the present disclosure.

One or more specific embodiments will be described below. In an effort to provide a concise description of these embodiments, all features of an actual implementation may not be described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments of the present subject matter, the articles "a," "an," "the," and "said" are intended to mean that there are one or more of the elements. The terms "comprising," "including," and "having" are intended to be inclusive and mean that there may be additional elements other than the listed elements. Furthermore, any numerical examples in the following discussion are intended to be non-limiting, and thus additional numerical values, ranges, and percentages are within the scope of the disclosed embodiments.

Embodiments of a hybrid switch for an inverter (e.g., super-resonant inverter) for a computed tomography (CT) system are provided. The hybrid switch includes a silicon carbide metal-oxide-semiconductor field-effect transistor (SiC MOSFET) portion and an insulated-gate bipolar transistor (IGBT) portion. The SiC MOSFET portion has a current rating lower than the IGBT portion of the hybrid switch. For the example, the SiC MOSFET portion and the IGBT portion may include current ratings of 120 Amperes (A) and 300 A, respectively. The power sides of both the SiC MOSFET portion and the IGBT portion are connected (electrically connected) together. In addition, the SiC MOSFET portion and IGBT portions each include a gate that enables independent (and separate) control of the respective portions of the hybrid switch. The portions of the hybrid switch may be utilized differently depending on the amount of power to be made and a frequency by the X-ray generation system (e.g., CT system). For example, in a high power (e.g., approximately 80 to 100 kilowatts (kW) or greater) range/low frequency (e.g., approximately 70 to 100 kilohertz (kHz)) range, the IGBT portion of the hybrid switch is mainly utilized. In a low power (e.g., approximately 20 to 40 kW) range/ high frequency (e.g., approximately 180 to 200 kHz) range, the SiC MOSFET portion of the hybrid switch is utilized. In a medium power (e.g., between approximately 40 and 80 kW) range/medium frequency (e.g., between approximately 100 to 180 kHz) range both the SiC MOSFET portion and the IGBT portion are utilized.

In the medium power/medium frequency range, the SiC MOSFET portion helps the commutation of the IGBT portion. For example in the medium power/medium frequency range, during commutation of the hybrid switch, the IGBT portion is turned on and an entirety of an electrical current flows through it. Subsequently, the SiC MOSFET portion is switched on but most of the current still passes through the IGBT portion due to its low RDS₍ₒₙ₎ (i.e., relative to the SiC MOSFET portion). Then the IGBT portion is switched off, which causes the electrical current to pass through the SiC MOSFET. Eventually, the tail current from the IGBT dies out while the electrical current is flowing through the SiC MOSFET. Then, the SiC MOSFET is switched off. This configuration utilizes the SiC MOSFET for a short period of time. Control of the hybrid switch in the described manner keeps conduction losses low. The tail current does not add to the switching losses since the voltage across the switch is zero. As a result, both switching losses and conduction losses are minimized when utilizing the hybrid switch.

Utilization of the hybrid switch in the inverter may increase generator (e.g., X-ray generator) power capability, while reducing the footprint and cost of the main inverter. In addition, utilization of the hybrid switch improves the assembly of the main inverter by reducing the number of mechanical parts. Further, the reduction in the number of components in the main inverter improves the inverter's reliability.

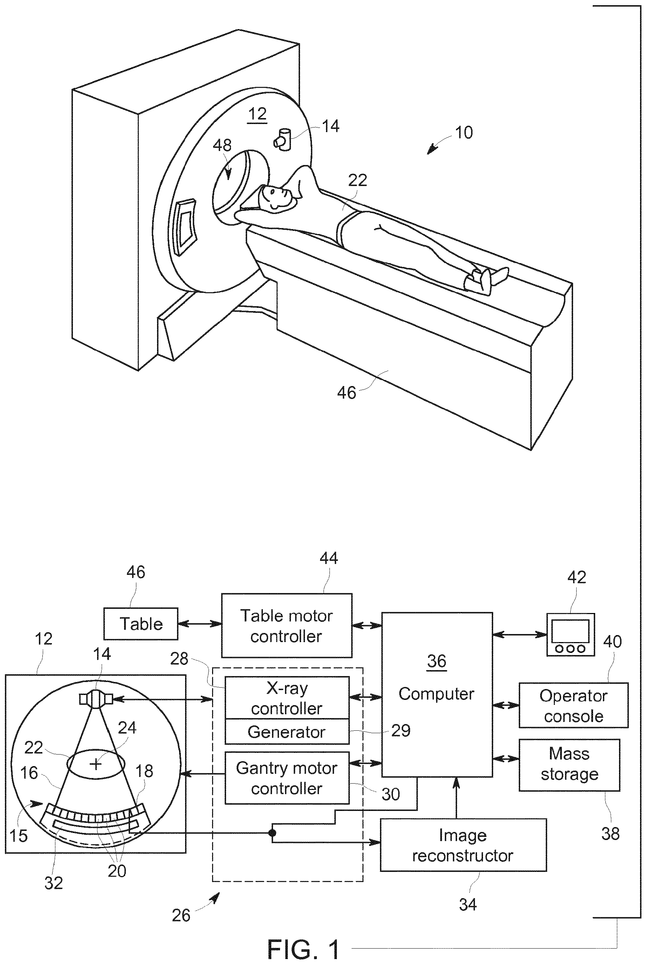

Although the following embodiments are discussed in terms of a computed tomography (CT) imaging system, the embodiments may also be utilized with other imaging systems (e.g., PET, CT/PET, SPECT, nuclear CT, etc.). With the preceding in mind and referring to FIG. 1, a CT imaging system 10 is shown, by way of example. The CT imaging system includes a gantry 12. The gantry 12 has an X-ray source 14 that projects a beam of X-rays 16 toward a detector assembly 15 on the opposite side of the gantry 12. The detector assembly 15 includes a collimator assembly 18, a plurality of detector modules 20, and data acquisition systems (DAS) 32. The plurality of detector modules 20 detect the projected X-rays that pass through a patient 22, and DAS 32 converts the data to digital signals for subsequent processing. Each detector module 20 in a conventional system produces an analog electrical signal that represents the intensity of an incident X-ray beam and hence the attenuated beam as it passes through the patient 22. During a scan to acquire X-ray projection data, gantry 12 and the components mounted thereon rotate about a center of rotation 24 so as to collect attenuation data from a multitude of view angles relative to the imaged volume.

Rotation of gantry 12 and the operation of X-ray source 14 are governed by a control mechanism 26 of CT system 10. Control mechanism 26 includes an X-ray controller 28 and a generator 29 (e.g., X-ray generation system 50 described below) that provides power and timing signals to an X-ray source 14 and a gantry motor controller 30 that controls the rotational speed and position of gantry 12. An image reconstructor 34 receives sampled and digitized X-ray data from DAS 32 and performs high-speed reconstruction. The reconstructed image is applied as an input to a computer 36, which stores the image in a mass storage device 38. Computer 36 also receives commands and scanning parameters from an operator via console 40. An associated display 42 allows the operator to observe the reconstructed image and other data from computer 36. The operator supplied commands and parameters are used by computer 36 to provide control signals and information to DAS 32, X-ray controller 28, and gantry motor controller 30. In addition, computer 36 operates a table motor controller 44, which controls a motorized table 46 to position patient 22 and gantry 12. Particularly, table 46 moves (e.g., extends) portions of patient 22 on the patient support through a gantry opening or bore 48.

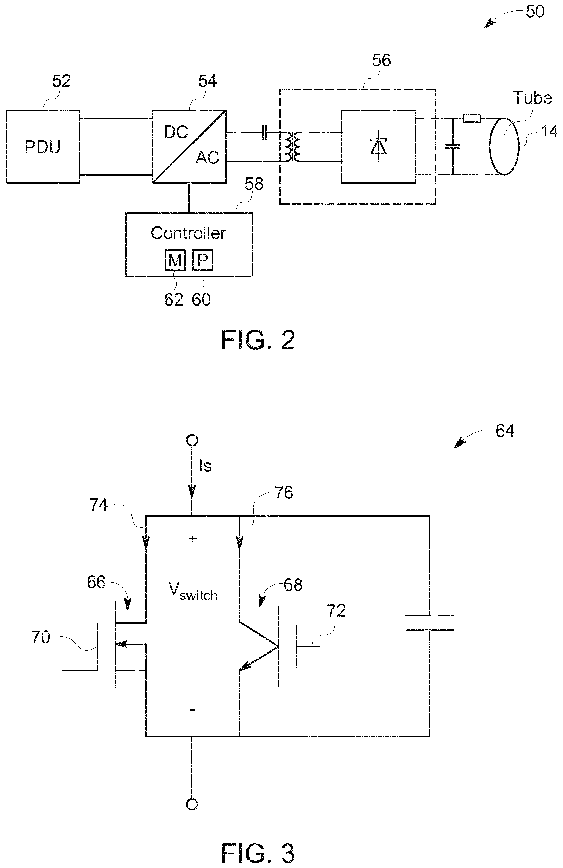

FIG. 2 is a schematic diagram of a portion (X-ray generation system 50) of the CT imaging system 10 of FIG. 1. The X-ray generation system 50 includes a power distribution unit (PDU) 52, an inverter 54, a high voltage (HV) tank 56, and the X-ray source 14 (e.g., X-ray tube). The PDU 52 provides a DC voltage, for example, 750 VDC, to the inverter 54. The inverter 54 generates an AC voltage waveform, for example, 300 VAC, at a specified frequency, e.g., 70 to 200 kHz. The AC waveform is fed to the HV tank 56, which has a transformer and rectifiers (not shown) that develop a DC HV potential. The HV potential is applied to the X-ray source. 14 (e.g., X-ray tube).

As described in greater detail below, the inverter 54 includes a single hybrid switch for generating the AC voltage waveform. The hybrid switch includes a single SiC MOSFET portion (e.g., having a high frequency capability) and a single insulated-gate bipolar transistor (IGBT) portion (e.g., having a high current capability) to be utilized in a super resonant inverter topology. The configuration of the hybrid switch minimizes both switching losses and conduction losses during switch utilization. Utilization of the hybrid switch in the inverter 54 may increase generator (e.g., X-ray generator) power capability, while reducing the footprint and cost of the inverter 54. In addition, utilization of the hybrid switch improves the assembly of the inverter 54 by reducing the number of mechanical parts. Further, the reduction in the number of components in the inverter 54 improves the inverter's reliability.

The inverter 54 (including the hybrid switch) is controlled by a controller 58 having a processor 60 or multiple processors and a memory 62. The controller 58 independently controls the SiC MOSFET portion and the IGBT portion of the hybrid switch. In certain embodiments, the controller 58 may control the SiC MOSFET portion and the IGBT portion differently based on a power and frequency utilized by the X-ray generation system 50 (e.g., of the CT system 10). The processor 60 may be operatively coupled to the memory 62 to execute instructions for carrying out the presently disclosed techniques. These instructions may be encoded in programs or code stored in a tangible non-transitory computer-readable medium, such as the memory 62 and/or other storage. The processor 60 may be a general purpose processor (e.g., processor of a desktop/laptop computer), system-on-chip (SoC) device, or some other processor configuration. In certain embodiments, instead of the processor 60, application-specific integrated circuits may be utilized. The memory 62, in the embodiment, includes a computer readable medium, such as, without limitation, a hard disk drive, a solid state drive, diskette, flash drive, a compact disc, a digital video disc, random access memory (RAM), and/or any suitable storage device that enables the processor 60 to store, retrieve, and/or execute instructions and/or data. The memory 62 may include one or more local and/or remote storage devices.

FIG. 3 is a circuit diagram of an embodiment of a hybrid switch 64 for the inverter 54 of the CT imaging system 10 of FIGS. 1 and 2. The hybrid switch 64 includes the SiC MOSFET portion 66 and the IGBT portion 68. The SiC MOSFET portion 66 includes a current rating lower than the IGBT portion 68 of the hybrid switch 64. For the example, the SiC MOSFET portion 66 and the IGBT portion 68 may include current ratings of 120 Amperes (A) and 300 A, respectively. The SiC MOSFET portion 66 and the IGBT portion 68 are associated with gates 70, 72, respectively. The gates 70, 72 enable the controller 58 to independently and separately control which portions 66, 68 of the hybrid switch 64 to utilize during operation of the switch by turning on (conducting state) and off (non-conducting state) the switches 66, 68. The SiC MOSFET portion 66 and the IGBT portion 68 include a respective power side 74, 76 that are coupled together to receive an electrical current, Is, when a voltage, V_{switch}, is applied across the hybrid switch 64. When the IGBT portion 68 is on and the SiC MOSFET portion 66 is off, an entirety of the current flows through the IGBT portion 68. When both the IGBT portion 68 and the SiC MOSFET portion 66 are on, most of the current flows through the IGBT portion 68 due to its low on resistance, RDS₍ₒₙ₎ (i.e., relative to the SiC MOSFET portion 66). When the SiC MOSFET portion 66 is on and the IGBT portion 68 is off, an entirety of the current flows through the SiC MOSFET portion 66.

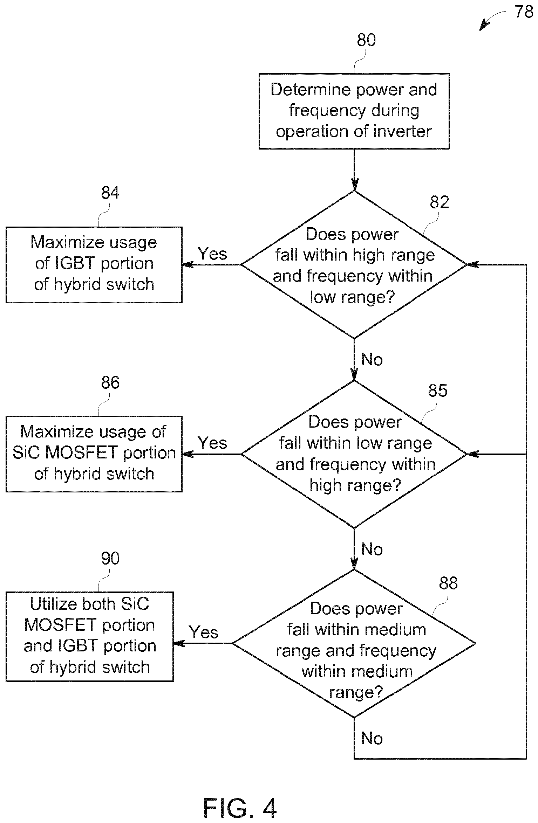

Based on the frequency and the power utilized during the operation of the inverter 54, the controller 58 may utilize different 66, 68 portions of the hybrid switch 64. FIG. 4 is a flow chart of a method 78 for utilizing the hybrid switch 64 of the inverter 54 for the CT imaging system 10. One or more of the steps of the method 78 may be performed by the controller 58. Some of the steps of the method 78 may be performed simultaneously or in a different order from that illustrated in FIG. 4. The method 78 includes determining a power and a frequency utilized during operation of the inverter 54 (and the CT imaging system 30 and the X-ray generation system 50) (block 80). In certain embodiments, the power may range between approximately 20 and 100 kW or greater. A low range for power may range between approximately 20 20 kW and 40 kW. A medium range for power may range between approximately 40 and 80 kW. A high range for power may range between approximately 80 and 100 kW or greater. In certain embodiments, the frequency may range between approximately 70 and 200 kHz. A low range for frequency may range between approximately 70 and 100 kHz. A medium range for frequency may range between approximately 100 and 180 kHz. A high range for frequency may between approximately 180 and 200 kHz. Typically, with a super-resonant converter, the maximal power is made at a low frequency and the minimum power at a high frequency.

Based on the determination of the power and the frequency, the method 78 includes independently controlling which portion 66, 68 of the hybrid switch 64 to utilize based on the power and the frequency. Specifically, the method 78 includes determining whether the power falls within the high range and the frequency within the low range (block 82). If the power falls within the high range and the frequency with the low range, the method 78 includes maximizing usage of the IGBT portion 68 (e.g., solely utilizing or utilizing a majority of the time) of the hybrid switch 64 (block 84). If the power does not fall within the high range and the frequency does not fall within the low range, the method 78 includes determining whether the power falls within the low range and the frequency within the high range (block 85). If the power falls within the low range and the frequency within the high range, the method 78 includes maximizing usage of the SiC MOSFET portion 66 (e.g., solely utilizing or utilizing a majority of the time) of the hybrid switch 64 (block 86). If the power does not fall within the low range and the frequency within the high range, the method 78 includes determining whether the power falls within the medium range and the frequency within the medium range (block 88). If the power falls within the medium range and the frequency within the medium range, the method 78 includes utilizing both the SiC MOSFET portion 66 and the IGBT portion 68 (block 90). In particular, the SiC MOSFET 66 helps in the commutation of the IGBT portion 68 to minimize switching losses and conduction losses. If the power does not within the medium range and the frequency within the medium range, the method 78 includes determining what ranges the power and the frequency fall under.

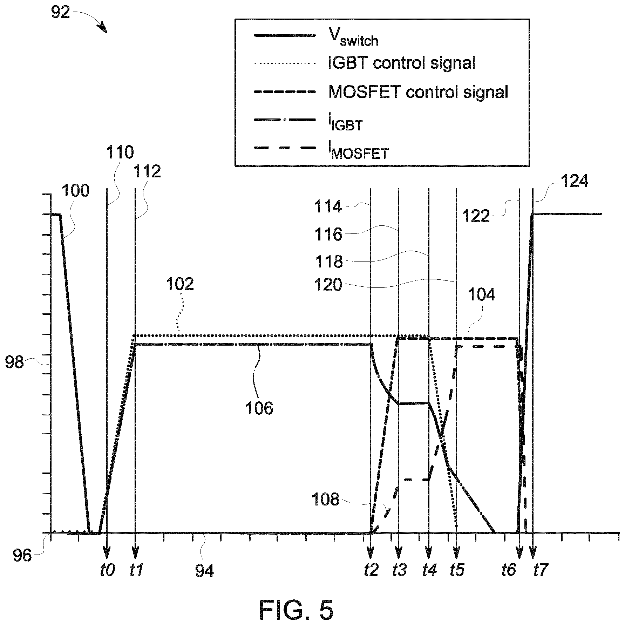

As noted above, if the power falls within the medium range and the frequency within the medium range, both portions 66, 68 of the hybrid switch 64 are utilized. FIG. 5 is a graph 92 of timing for commutation of the hybrid switch 64 in accordance with an embodiment of the present disclosure (e.g., when the power falls within the medium range and the frequency within the medium range). The horizontal axis 94 of the graph 92 represents time. The intersection 96 of the vertical axis 98 with the horizontal axis 94 of the graph 92 represents a zero stage (e.g., for voltage, current, absence of command signal, etc.). The line 100 (shown as a solid line) represents the voltage, V_{switch}, applied to the hybrid switch 64. The line 102 (shown as a dotted line) represents the control signal provided to the IGBT portion 68 of the hybrid switch 64. The line 104 (shown as a dashed line) represents the control signal provided to the SiC MOSFET portion 66 of the hybrid switch 64. The line 106 (shown as a dotted-dash line) represents the current, I_{IGBT}, flowing through the IGBT portion 68 of the hybrid switch 64. The line 108 (shown as the long dash line) represents the current, I_{MOSFET}, flowing through the SiC MOSFET portion 66 of the hybrid switch 64. The arrows 110, 112, 114, 116, 118, 120, 122, and 124 represent time points, t0, t1, t2, t3, t4, t5, t6, and t7, respectively.

The voltage, V_{switch} (as shown by line 100), applied across the switch is zero during commutation of the hybrid switch 62 from prior to t0 to t7. Near t0, the IGBT portion 68 is turned on (as indicated by line 102) and current, I_{IGBT} (as shown by line 106), flows through the IGBT portion 68. From t0 to t2, only the IGBT portion 68 is turned on and all of the current flows through portion 68. At t2, the SiC MOSFET portion 66 is turned on (as indicated by line 104) but most of the current initially still passes through the IGBT portion 68 due its low RDS₍ₒₙ₎ (i.e., relative to the SiC MOSFET portion 66). The IGBT portion 68 is switched off at t3, which causes most of the current to pass through the SiC MOSFET portion 66 as indicated line 108. The IGBT portion 68 has a tail current that dies out between t5 and t6 and produces no switching losses. At t5, the SiC MOSFET portion 66 is switched off. The SiC MOSFET portion 66 is utilized for a short period of time (e.g., relative to the time the IGBT portion 68 of the hybrid switch 64 is utilized). As a result, conduction losses are low or minimal. The tail current does not add to the switching losses since the voltage, V_{switch}, applied across the hybrid switch 64 during commutation is zero. Thus, both switching losses and conduction losses are minimized during use of the hybrid switch 64.

Various technical effects of the disclosed embodiments of the hybrid switch 64 for the inverter 54 may include increasing generator (e.g., X-ray generator) power capability, while reducing the footprint and cost of the main inverter 54. In addition, utilization of the hybrid switch 64 improves the assembly of the main inverter 54 by reducing the number of mechanical parts. Further, the reduction in the number of components in the main inverter 54 improves the inverter's reliability.

This written description uses examples to disclose the subject matter, including the preferred mode, and also to enable any person skilled in the art to practice the subject matter, including making and using any devices or systems and performing any incorporated methods. The patentable scope of the subject matter is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

Various aspects and embodiments of the present invention are defined by the following numbered clauses:
1. An X-ray generation system, comprising:
   an X-ray source;
   a high voltage tank coupled to the X-ray source;
   an inverter coupled to the high voltage tank, wherein the inverter comprises:
      a hybrid switch comprising a silicon carbide metal-oxide-semiconductor field-effect transistor (SiC MOSFET) portion and an insulated-gate bipolar transistor (IGBT) portion; and
   a controller programmed to control commutation of the hybrid switch based on a frequency and a power utilized by the X-ray generation system.
2. The X-ray generation system of clause 1, wherein the controller is programmed to independently control the SiC MOSFET portion and the IGBT portion of the hybrid switch.
3. The X-ray generation system of any preceding clause, wherein the hybrid switch comprises a first gate associated with the SiC MOSFET portion and a second gate associated with the IGBT portion to enable the independent control by the controller.
4. The X-ray generation system of any preceding clause, wherein the controller is programmed to utilize the SiC MOSFET portion and the IGBT portion differently based on the frequency and the power utilized by the X-ray generation system.
5. The X-ray generation system of any preceding clause, wherein, when the frequency is in a low frequency range and the power is in a high power range, the controller is programmed to utilize the IGBT portion.
6. The X-ray generation system of any preceding clause, wherein, when the frequency is in a high frequency range and the power is in a low power range, the controller is programmed to utilize SiC MOSFET portion.
7. The X-ray generation system of any preceding clause, wherein, when the frequency is in a medium frequency range and the power is in a medium power range, the controller is programmed to utilize both the IGBT portion and the SiC MOSFET portion.
8. The X-ray generation system of any preceding clause, wherein, when the frequency is in the medium frequency range and the power is in the medium power range, the controller is programmed in a sequential order to turn on only the IGBT portion to enable an entirety of the electrical current to flow through the IGBT portion, turn on the SiC MOSFET portion where most of the electrical current still flows through the IGBT portion, to switch off the IGBT portion to enable the entirety of the electrical current to flow through the SiC MOSFET portion, and to switch off the SiC MOSFET portion.
9. The X-ray generation system of any preceding clause, wherein the SiC MOSFET portion has a lower current rating than the IGBT portion.
10. The X-ray generation system of any preceding clause, wherein a respective power side of the SiC MOSFET portion and the IGBT portion are coupled together.
11. The X-ray generation system of any preceding clause, wherein the X-ray generation system is configured to be utilized with a computed tomography (CT) system.
12. A method for utilizing an inverter of a computed tomography (CT) system, comprising:
   determining, via a controller, a power and a frequency for operation of the inverter, wherein the inverter comprises a hybrid switch comprising a silicon carbide metal-oxide-semiconductor field-effect transistor (SiC MOSFET) portion and an insulated-gate bipolar transistor (IGBT) portion, and the SiC MOSFET portion has a lower current rating than the IGBT portion; and
   independently controlling, via the controller, which portion of the hybrid switch to utilize based on the power and the frequency.
13. The method of any preceding clause, comprising, when the frequency is in a low frequency range and the power is in a high power range, utilizing the IGBT portion of the hybrid switch via the controller.
14. The method of any preceding clause, comprising, when the frequency is in a high frequency range and the power is in a low power range, utilizing the SiC MOSFET portion of the hybrid switch via the controller.
15. The method of any preceding clause, comprising, when the frequency is in a medium frequency range and the power is in a medium power range, utilizing both the IGBT portion and the SiC MOSFET portion of the hybrid switch via the controller.
16. The method of any preceding clause, comprising, when the frequency is in a medium frequency range and the power is in a medium power range, sequentially, via the controller, turning on only the IGBT portion to enable an entirety of the electrical current to flow through the IGBT portion, turning on the SiC MOSFET portion where most of the electrical current still flows through the IGBT portion, switching off the IGBT portion to enable the entirety of the electrical current to flow through the SiC MOSFET portion, and switching off the SiC MOSFET portion.
17. An inverter for a computed tomography (CT) system, comprising:
   a hybrid switch comprising:
      a silicon carbide metal-oxide-semiconductor field-effect transistor (SiC MOSFET) portion;
      an insulated-gate bipolar transistor (IGBT) portion;
      a first gate associated with the SiC MOSFET portion; and
      a second gate associated with the IGBT portion;
   wherein the SiC MOSFET portion and the IGBT portion of the hybrid switch are configured to be independently controlled via the first gate and the second gate.
18. The inverter of any preceding clause, wherein the SiC MOSFET portion has a lower current rating than the IGBT portion.
19. The inverter of any preceding clause, wherein a respective power side of the SiC MOSFET portion and the IGBT portion are coupled together.
20. The inverter of any preceding clause, wherein the SiC MOSFET portion and the IGBT portion are configured to be controlled differently based on a frequency and a power utilized by the CT system.

## Claims

1. An X-ray generation system (50), comprising:
an X-ray source (14);
a high voltage tank (56) coupled to the X-ray source (14);
an inverter (54) coupled to the high voltage tank (56), wherein the inverter (54) comprises:
a hybrid switch (64) comprising a silicon carbide metal-oxide-semiconductor field-effect transistor (SiC MOSFET) portion (66) and an insulated-gate bipolar transistor (IGBT) portion (68); and
a controller (58) programmed to control commutation of the hybrid switch (64) based on a frequency and a power utilized by the X-ray generation system (50).

2. The X-ray generation system (50) of claim 1, wherein the controller (58) is programmed to independently control the SiC MOSFET portion (66) and the IGBT portion (68) of the hybrid switch (64).

3. The X-ray generation system (50) of any preceding claim, wherein the hybrid switch (64) comprises a first gate (70) associated with the SiC MOSFET portion (66) and a second gate (72) associated with the IGBT portion (68) to enable the independent control by the controller (58).

4. The X-ray generation system (50) of any preceding claim, wherein the controller (58) is programmed to utilize the SiC MOSFET portion (66) and the IGBT portion (68) differently based on the frequency and the power utilized by the X-ray generation system (50).

5. The X-ray generation system (50) of any preceding claim, wherein, when the frequency is in a low frequency range and the power is in a high power range, the controller (58) is programmed to utilize the IGBT portion (68).

6. The X-ray generation system (50) of any preceding claim, wherein, when the frequency is in a high frequency range and the power is in a low power range, the controller (58) is programmed to utilize SiC MOSFET portion (66).

7. The X-ray generation system (50) of any preceding claim, wherein, when the frequency is in a medium frequency range and the power is in a medium power range, the controller (58) is programmed to utilize both the IGBT portion (66) and the SiC MOSFET portion (68).

8. The X-ray generation system (50) of claim 7, wherein, when the frequency is in the medium frequency range and the power is in the medium power range, the controller (58) is programmed in a sequential order to turn on only the IGBT portion (66) to enable an entirety of the electrical current to flow through the IGBT portion (68), turn on the SiC MOSFET portion (68) where most of the electrical current still flows through the IGBT portion (68), to switch off the IGBT portion (68) to enable the entirety of the electrical current to flow through the SiC MOSFET portion (66), and to switch off the SiC MOSFET portion (66).

9. The X-ray generation system (50) of any preceding claim, wherein the SiC MOSFET portion (66) has a lower current rating than the IGBT portion (68).

10. The X-ray generation system (50) of any preceding claim, wherein a respective power side of the SiC MOSFET portion (66) and the IGBT portion (68) are coupled together.

11. The X-ray generation system (50) of any preceding claim, wherein the X-ray generation system (50) is configured to be utilized with a computed tomography (CT) system (10).

12. A method (78) for utilizing an inverter (54) of a computed tomography (CT) system (10), comprising:
determining (80), via a controller (58), a power and a frequency for operation of the inverter (54), wherein the inverter (54) comprises a hybrid switch (64) comprising a silicon carbide metal-oxide-semiconductor field-effect transistor (SiC MOSFET) portion (66) and an insulated-gate bipolar transistor (IGBT) portion (68), and the SiC MOSFET portion (66) has a lower current rating than the IGBT portion (68); and
independently controlling, via the controller (58), which portion of the hybrid switch (64) to utilize based on the power and the frequency.

13. The method (78) of claim 12, comprising, when the frequency is in a low frequency range and the power is in a high power range, utilizing (85) the IGBT portion (68) of the hybrid switch (64) via the controller (58).

14. The method (78) of claim 12 or claim 13, comprising, when the frequency is in a high frequency range and the power is in a low power range, utilizing (82) the SiC MOSFET portion (66) of the hybrid switch (64) via the controller (58).

15. The method (78) of any of claims 12 to 14, comprising, when the frequency is in a medium frequency range and the power is in a medium power range, utilizing (88) both the IGBT portion (68) and the SiC MOSFET portion (66) of the hybrid switch (64) via the controller (58).
